# EUROPEAN PATENT APPLICATION

(11) **EP 2 143 382 A1**
(43) Date of publication of application: **13.01.2010**
(21) Application number: 09163974.0
(22) Date of filing: 29.06.2009
(51) Int. Cl.: A61B 8/00

(54) **Ultrasound System Having Virtual Keyboard and Method of Displaying the Same**

(30) Priority: 10.07.2008 KR 20080066835
(71) Applicant: MEDISON CO., LTD., Kangwon-do 250-870 (KR)
(72) Inventor: Shin, Soo Hwan, 135-851, Seoul (KR); Park, Sung In, 135-851, Seoul (KR); Lee, Su Myeong, 135-851, Seoul (KR)
(74) Representative: Schmid, Wolfgang

(57) **Abstract**

The present invention relates to an ultrasound system capable of displaying a virtual keyboard image on the touch screen and a method of displaying the virtual keyboard image on a part of a display screen of the display unit. The ultrasound system comprises: a control panel configured to receive user instructions for displaying a virtual keyboard image; a display unit configured to display the virtual keyboard image and to receive a command by touch type; and a processor unit coupled to the control panel and the display unit, the processor unit being configured to display an ultrasound image on the display unit to display the virtual keyboard image on a part of a display screen of the display unit.

## Description

The present application claims priority from Korean Patent Application No. 10-2008-0066835 filed on July 10, 2008, the entire subject matters of which are incorporated herein by reference.

### BACKGROUND OF THE INVENTION

### [Technical Field]

The present invention generally relates to ultrasound systems, and more particularly to an ultrasound system having a virtual keyboard and a method of displaying the virtual keyboard on a part of a display screen of the display unit.

### [Background Art]

A touch screen is widely used as a user interface in various imaging devices. The touch screen is a display that can detect the presence of a user's touch and a touched location in a display area. The imaging device may process previously set instructions in response to the detected presence or location. The touch screen may be manufactured by adding a touch panel on a screen of a general display such as a monitor. The touch panel may be segmented into a plurality of grids and an infrared sensor may be mounted on each of the grids. The infrared sensor may sense a temperature change so that it can be recognized whether a touch is present on the display area. The imaging device may recognize what the user selects based on a touch position on the display area of the touch screen and then generate input signals corresponding to the selection.

Generally, the ultrasound system has an alpha-numeric keyboard for character and number input. Although the alpha-numeric keyboard is not typically used, the alpha-numeric keyboard occupies a significant region on the control panel. Also, the alpha-numeric keyboard can be drawn out of the control panel. In such a case, the user must change his or her position and it is significantly more difficult to use the alpha-numeric keyboard.

### BWEF DESCRIPTION OF THE DRAWINGS

- FIG. 1: is a schematic diagram of an illustrative embodiment of an ultrasound system.
- FIG. 2: is a schematic diagram showing the first virtual keyboard image for inputting text and numerical information.
- FIG. 3: is a schematic diagram showing the second virtual keyboard image for inputting numerical information.
- FIG. 4: is a schematic diagram showing the third virtual keyboard image for inputting an ultrasound image scanning operation information.

### DETAILED DESCRIPTION OF THE INVENTION

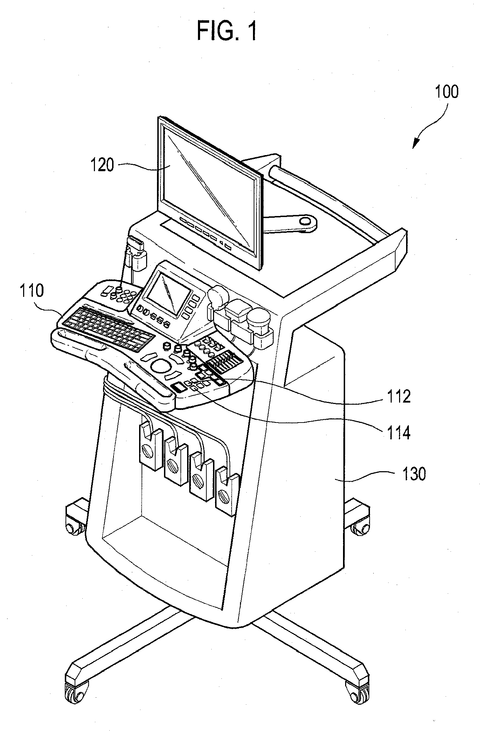

FIG. 1 is a schematic diagram of an illustrative embodiment of an ultrasound system. The ultrasound system 100 may include: a control panel 110 configured to receive user instructions for displaying a virtual keyboard image; a display unit 120 configured to display the virtual keyboard image thereon and to receive a command by touch type; and a processor unit (not shown) coupled to the control panel and the display unit, the processor unit being configured to display an ultrasound image on the display unit and to display the virtual keyboard image on a part of a display screen of the display unit 120.

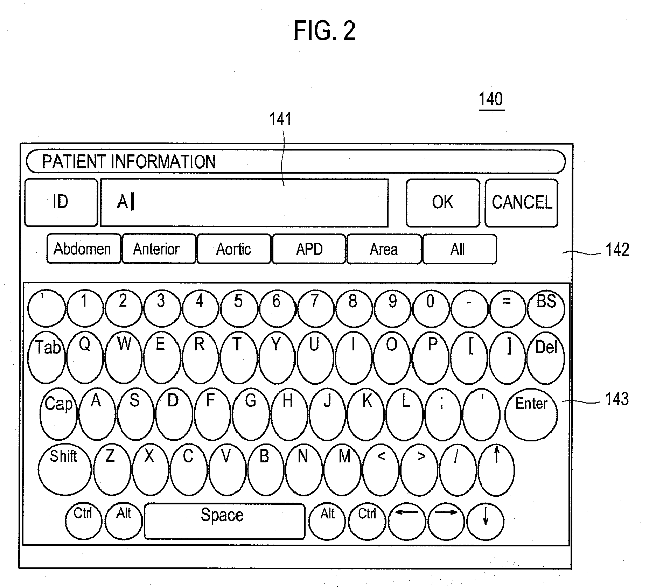

The control panel 110 may be configured to receive user instructions for displaying a virtual keyboard image on a part of a display screen of the display unit 120. For example, a user may input user instructions such as a virtual keyboard image display instruction by pressing a text button 112 and a virtual keyboard image change instruction by pressing virtual keyboard image change button 114. The virtual keyboard image is selected from a group of (i) a first image for inputting text and numerical information, (ii) a second image for inputting numerical information only, and (iii) a third image for inputting an ultrasound image scanning operation information. One of the first, second and third images (may be referred from Figs. 2 to. 4) may be displayed on the display unit 120 according to the user instructions. The processor unit of the ultrasound system 100 is configured to change from one image of the first, second and third images to another image according to the user instructions. For example, the first image turns to the second image when you press the virtual keyboard image change button 114 as a virtual keyboard change instruction. Further, the second image turns to the third virtual keyboard image when you press the virtual keyboard image change button 114 again.

The first virtual keyboard image 140 may include input confirmation region 141, reference words list region 142 and button region 143, as illustrated in Fig. 2. The words, which were received from the button region 143, may be displayed at the input confirmation region 141. The button region 143 may include a number of buttons for receiving the user instructions. Words buttons, which are related to the specific characters, may be displayed at the reference words list region 142. The user can recognize characters, which are received from the button region 143, due to displaying the characters on the input confirmation region 141. The buttons located in the button region 143 are shaped as lengthy oval. Also, the vertical length of the buttons is longer than the horizontal length of the buttons. The characters on the buttons are located at the upper region of the buttons. As such, the user can recognize the characters on the buttons while pressing the buttons.

The virtual keyboard image may be displayed on a part of a display screen of the display unit 120. The display unit 120 may be configured to display the virtual keyboard image thereon and to receive a command by touch type. The display unit 120 may be made from Liquid Crystal Display touch panel. Thus, the user can touch the display unit 120 to input the text information, numerical information and the ultrasound image scanning operation information.

The processor unit (not shown) is mounted on a body 130. The processor unit is coupled to the control panel 110 and the display unit 120. The processor unit is configured to display an ultrasound image on the display unit 120. Also, the processor unit is configured to display the virtual keyboard image on a part of a display screen of the display unit 120. For example, the processor unit may control the display unit 120 to display the virtual keyboard image 140 including the input confirmation region 141, the reference words list region 142 and the button region 143, as illustrated in Fig. 2, according to the user instructions. Namely, the processor unit may display the first virtual keyboard image on the display unit 120 when the user presses the text button 112. The pressing of the text button 112 is regarded as a first virtual keyboard display instruction. Also, the processor unit may display the first virtual keyboard image on the display unit 120 when the user moves a cursor to a specific part of the display unit 120. The moving of the cursor to the specific part of the display unit 120 is regarded as a first virtual keyboard display instruction.

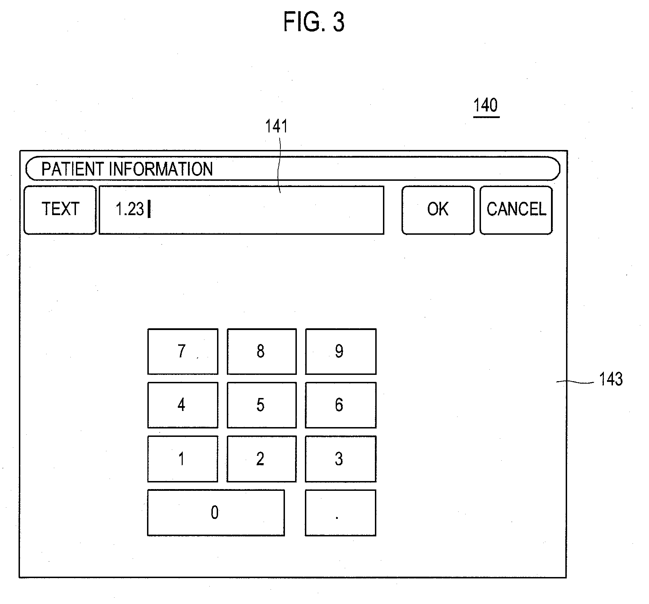

Also, the processor unit may control the display unit 120 to display the second virtual keyboard image 140 including the numeric buttons, as illustrated in Fig. 3, when the user selects the second virtual keyboard image. The processor unit is configured to display the second virtual keyboard image, as illustrated in Fig. 3, on the display unit 120 when the user presses the text button. Also, the processor unit is configured to display the second virtual keyboard image, as illustrated in Fig. 3, on the display unit 120 when the user moves the cursor to the specific part of the display unit 120. The moving of the cursor to the specific part of the display unit 120 is regarded as a second virtual keyboard display instruction. The numeric buttons, which are included in the second virtual keyboard image, are configured to set in an array similar to telephone buttons. Thus, the user can input the numbers faster.

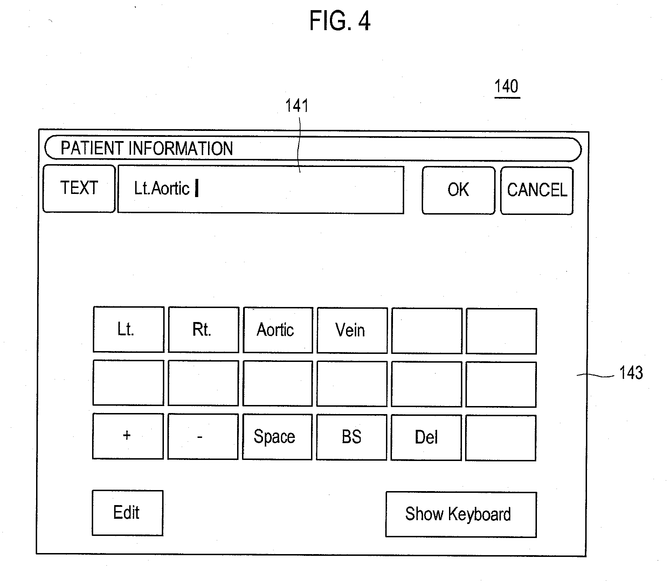

Also, the processor unit is configured to control the display unit 120 to display the virtual keyboard image for text input, which is related to the scanning operation as illustrated in the Fig. 4, when the user select the third virtual keyboard image. It is necessary to input a text on the ultrasound image for the ultrasound image explanation during the scanning operation. The text, which is used in the ultrasound image scanning operation, may be limited. The third virtual keyboard image may be displayed when the user presses the text button 112 during the scanning operation. The text made up of a character combination is typically used. The character combination may be displayed on the ultrasound image when the user presses a character combination button. The character combination may be edited by the user. Further, it may not be necessary to input a blank because the blank may be inputted along with the character combination when the user presses the character combination button. The first virtual keyboard image, as illustrated in Fig. 2, is displayed on the display unit 120 when the user presses the virtual keyboard image change button 114 as the virtual keyboard change instruction during displaying of the third virtual keyboard image on the display unit 120. Thus, the user can input the text easily.

It is important that changing of the virtual keyboard image may not mean changing of the virtual keyboard image in a regular sequence. The first virtual keyboard image may be displayed, as illustrated in Fig. 2, when the user presses the virtual keyboard image change button 114 during displaying of the third virtual keyboard image as illustrated in Fig. 4. Also, the third virtual keyboard image may be displayed when the user presses the virtual keyboard image change button 114 during displaying of the first virtual keyboard image.

The processor unit is configured to assess the input state and control the display unit 120 to display the adequate virtual keyboard image to the input state. The input state may include inputting text and numerical information, inputting numerical information only and inputting an ultrasound image scanning operation information.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a control panel configured to receive user instructions for displaying a virtual keyboard image;
a display unit configured to display the virtual keyboard image and to receive a command by touch type; and
a processor unit coupled to the control panel and the display unit, the processor unit being configured to display an ultrasound image on the display unit and to display the virtual keyboard image on a part of a display screen of the display unit.

2. The ultrasound system of Claim 1, wherein the virtual keyboard image includes a button region for displaying keyboard buttons; and
an input confirmation region for displaying information inputted through the keyboard buttons.

3. The ultrasound system of Claim 1, wherein the virtual keyboard image is selected from a group of (i) a first image for inputting text and numerical information, (ii) a second image for inputting numerical information only, and (iii) a third image for inputting an ultrasound image scanning operation information.

4. The ultrasound system of Claim 3, wherein the first image includes a reference words list region for displaying buttons of words related to characters inputted by a user.

5. The ultrasound system of Claim 3, wherein the processor unit is configured to change from one image of the first, second and third images to another image according to the user instructions.

6. A method of displaying a virtual keyboard image in an ultrasound system, comprising:
a) displaying an ultrasound image on a display unit within the ultrasound system;
b) receiving a command for displaying a virtual keyboard image from a control panel within the ultrasound system; and
c) displaying the virtual keyboard image on a part of a display screen of the display unit.

7. The method of Claim 6, wherein the virtual keyboard image includes a button region for displaying keyboard buttons; and
an input confirmation region for displaying information inputted through the keyboard buttons.

8. The method of Claim 6, wherein the virtual keyboard image is selected from a group of (i) a first image for inputting text and numerical information, (ii) a second image for inputting numerical information only, and (iii) a third image for inputting an ultrasound image scanning operation information.

9. The method of Claim 8, wherein the step c) includes;
changing from one image of the first, second and third images to another image.
